# EUROPEAN PATENT APPLICATION

(11) **EP 4 796 207 A1**
(43) Date of publication of application: **26.08.2026**
(21) Application number: 24913818.1
(22) Date of filing: 17.12.2024
(51) Int. Cl.: B01D 53/86, B01D 46/00, B01J 23/16, B01J 23/10, B01J 35/61, B01J 38/00, F24F 8/167

(54) **REFRIGERATOR**

(30) Priority: 28.12.2023 JP 2023222551
(71) Applicant: Samsung Electronics Co., Ltd., Suwon-si, Gyeonggi-do 16677 (KR)
(72) Inventor: IWAMOTO, Kiyoshi, Yokohama-shi, Kanagawa 230-0027 (JP); MURATA, Kenichi, Yokohama-shi, Kanagawa 230-0027 (JP); KUBO, Kosuke, Yokohama-shi, Kanagawa 230-0027 (JP); OKAZAKI, Takao, Tsu-shi, Mie 514-8507 (JP)
(74) Representative: Walaski, Jan Filip
(86) International application number: PCT/KR2024/097080
(87) International publication number: WO 2025/144014

(57) **Abstract**

Provided is a refrigerator in which a deodorizing filter can exhibit excellent deodorizing performance for a long period of time. Provided is a refrigerator comprising: an inner case forming a storage chamber; a body including an outer case as an outer layer of the inner case; a door configured to open and close the storage chamber; and a deodorizing device disposed on the inner surface of the storage chamber, wherein the deodorizing device includes a deodorizing filter, the deodorizing filter includes a substrate and a deodorizing film on the surface of the substrate, the deodorizing film contains a deodorizing catalyst, and the deodorizing catalyst includes δ-type MnO₂ and CeO₂, and has a structure in which CeO₂ particles are covered with δ-type MnO₂, the CeO₂-covering δ-type MnO₂ having an average particle size of 20 µm or less, and having a specific surface area of at least 250m²/g in terms of BET value.

## Description

### Technical Field

The present disclosure relates to a refrigerator including a deodorizing catalyst, a deodorizing film, and a deodorizing filter that are configured to adsorb and decompose odor molecules.

### Background Art

Using a deodorizing catalyst, which is prepared by mixing δ-type manganese dioxide (MnO₂) and cerium oxide (CeO₂), as a deodorizing filter used in conventional air purifiers or refrigerators is, for example, known in Patent Document 1.

### [Prior Art Document]

### [Patent Document]

Patent Document 1: JP 2021-130104

### Disclosure of Invention

### Technical Problem

δ-type MnO₂ has a layered structure, and for example, as compared with α-type MnO₂ having a tunnel-type structure with a small pore diameter, may have a property that facilitates adsorption of malodor molecules such as formaldehyde. Such δ-type MnO₂ exhibits a deodorizing function (catalytic function) of oxidizing and decomposing adsorbed malodor molecules. In addition, CeO₂ functions as a cocatalyst that regenerates catalytic functions of δ-type MnO₂ by supplying oxygen to the δ-type MnO₂. Such a cocatalyst is a material that is added to a main catalyst to improve activity, selectivity, and stability of the main catalyst. For example, δ-type MnO₂ is the main catalyst, and CeO₂ is the cocatalyst.

When applied to a deodorizing filter, there is a need for a deodorizing catalyst to exhibit superior deodorization performance over a longer period of time.

The present disclosure has been made to address the above issues, and a main objective of the present disclosure is to provide a refrigerator capable of exhibiting excellent deodorization performance over a long period of time through a deodorizing filter used in the refrigerator.

### Solution to Problem

A home appliance, such as a refrigerator, an air purifier, and an air conditioner, according to the present disclosure is as follows.

According to an embodiment, there is provided a refrigerator including: an inner case forming a storage chamber; a main body including an outer case as an outer layer of the inner case; a door configured to open and close the storage chamber; and a deodorizing device arranged on an inner surface of the storage chamber, wherein the deodorizing device includes a deodorizing filter, the deodorizing filter includes: a substrate; and a deodorizing film on a surface of the substrate, the deodorizing film includes a deodorizing catalyst, and the deodorizing catalyst contains δ-type MnO₂ and CeO, has a structure in which the CeO is coated with the δ-type MnO₂, and has an average particle diameter of the catalyst having the structure of 20 µm or less.

According to another embodiment, there is provided the refrigerator having a specific surface area of 250 m²/g or more as a BET value.

According to another embodiment, there is provided the refrigerator in which a content of the δ-type MnO₂ is 90 wt% to 99.5 wt%, and a content of the CeO₂ is 0.5 wt% to 10 wt%.

According to another embodiment, there is provided the refrigerator in which an average particle diameter of the δ-type MnO₂ coating the CeO₂ is 20 µm or less.

According to another embodiment, there is provided the refrigerator in which the δ-type MnO₂ has a layered structure, and a distance between adjacent layers included in the layered structure is 7 Å or more.

According to another embodiment, there is provided the refrigerator in which the deodorizing film further contains an inorganic binder.

According to another embodiment, there is provided the refrigerator in which the deodorizing film further contains activated carbon.

According to another embodiment, there is provided the refrigerator in which the deodorizing film further contains at least one selected from MgO, FeO, Fe₂O₃, ZrO₂, CuO, NiO, Mn₃O₄, Co₃O₄, Al₂O₃, Y₂O₃, ZnO, MoO₃, IrO₂, WO₃, W₂O₃, and TiO₂.

According to another embodiment, there is provided the refrigerator in which the substrate has a honeycomb structure.

According to another embodiment, there is provided the refrigerator further including an ultraviolet light source for regenerating the deodorizing catalyst.

According to an embodiment, there is provided an air purifier including: a main body having an inlet and an outlet; a fan configured to transfer air introduced through the inlet, to discharge the air through the outlet; and a deodorizing device disposed between the inlet and the outlet, wherein the deodorizing device includes a deodorizing filter, the deodorizing filter includes: a substrate; and a deodorizing film on a surface of the substrate, the deodorizing film includes a deodorizing catalyst, and the deodorizing catalyst contains δ-type MnO₂ and CeO₂, has a structure in which the CeO₂ is coated with the δ-type MnO₂, has an average particle diameter of the δ-type MnO₂ of 20 µm or less, and has a specific surface area of 250 m²/g or more as a BET value.

According to another embodiment, there is provided the air purifier in which a content of the δ-type MnO₂ is 90 wt% to 99.5 wt%, and a content of the CeO₂ is 0.5 wt% to 10 wt%.

According to another embodiment, there is provided the air purifier in which the average particle diameter of the δ-type MnO₂ coating the CeO₂ is 20 µm or less.

According to another embodiment, there is provided the air purifier, wherein the deodorizing film further contains an inorganic binder.

According to another embodiment, there is provided the air purifier, wherein the deodorizing film further contains activated carbon.

According to another embodiment, there is provided the air purifier, wherein the deodorizing film further contains at least one selected from MgO, FeO, Fe₂O₃, ZrO₂, CuO, NiO, Mn₃O₄, Co₃O₄, Al₂O₃, Y₂O₃, ZnO, MoO₃, IrO₂, WO₃, W₂O₃, and TiO₂.

According to another embodiment, there is provided the air purifier, wherein the substrate has a honeycomb structure.

According to an embodiment, there is provided a home appliance including: a main body including an inlet and an outlet; a fan configured to transfer air introduced through the inlet, to discharge the air through the outlet; a heat exchanger configured to exchange heat between air introduced through the inlet and a refrigerant; and a deodorizing device arranged between the inlet and the outlet, wherein the deodorizing device includes a deodorizing filter, the deodorizing filter includes: a substrate; and a deodorizing film on a surface of the substrate, the deodorizing film includes a deodorizing catalyst, and the deodorizing catalyst contains δ-type MnO₂ and CeO₂, has a structure in which the CeO₂ is coated with the δ-type MnO₂, has an average particle diameter of the δ-type MnO₂ of 20 µm or less, and has a specific surface area of 250 m²/g or more as a BET value.

### Advantageous Effects of Invention

According to the present disclosure configured as described above, provided is a refrigerator capable of exhibiting excellent deodorization performance over a long period of time through a deodorizing filter used in the refrigerator.

According to the present disclosure configured as described above, provided is an air purifier capable of exhibiting excellent deodorization performance over a long period of time through a deodorizing filter used in the air purifier.

According to the present disclosure configured as described above, provided is a home appliance capable of exhibiting excellent deodorization performance over a long period of time through a deodorizing filter used in the home appliance.

### Brief Description of Drawings

FIG. 1 is a perspective view of a refrigerator according to an embodiment of the present disclosure.
FIG. 2 is a front view illustrating the interior of a refrigerator according to an embodiment of the present disclosure.
FIG. 3 is a cross-sectional view of a refrigerator according to an embodiment of the present disclosure.
FIG. 4 is a perspective view illustrating the exterior of an air purifier according to an embodiment of the present disclosure.
FIG. 5 is a cross-sectional view showing the interior of an air purifier according to an embodiment of the present disclosure.
FIG. 6 is a perspective view showing the exterior of an air conditioner according to an embodiment of the present disclosure.
FIG. 7 is a cross-sectional view showing the interior of an air conditioner according to an embodiment of the present disclosure.
FIG. 8 is a view showing structures of a deodorizing filter, a deodorizing film, and a deodorizing catalyst, according to the present embodiment.
FIG. 9 shows an SEM image obtained by observing a deodorizing catalyst of the present embodiment (Example 4 in Experimental Example 1).
FIG. 10 shows EDX mapping performed on a deodorizing catalyst of the present embodiment (Example 4 in Experimental Example 1).
FIG. 11 shows an SEM image of a surface of a deodorizing catalyst of Comparative Example 4 in Experimental Example 1.
FIG. 12 shows EDX mapping performed on a deodorizing catalyst of Comparative Example 4 in Experimental Example 1.
FIG. 13 is a reaction scheme for a deodorizing catalyst of the present embodiment with respect to malodor components.
FIG. 14 is a diagram explaining a novel scientific discovery relating to decomposition of malodor molecules by a deodorizing catalyst of the present disclosure.
FIG. 15 is a diagram explaining test conditions, test results, and the like for Experimental Example 2.
FIG. 16 is a diagram explaining test conditions, test results, and the like for Experimental Example 3.
FIG. 17 is a diagram explaining the principle of promoting regeneration of a catalyst by UV irradiation.

### Best Mode for Carrying out the Invention

Various embodiments of the present disclosure and the terms used herein are not intended to limit the technical features described in the present disclosure to particular embodiments, and should be understood as including various changes, equivalents, or substitutes of the respective embodiments.

In connection with the description of the drawings, similar reference numerals may be used for similar or related elements.

A singular form of a noun corresponding to an item may include one item or a plurality of items, unless the relevant context clearly indicates otherwise.

In the present disclosure, each of phrases such as "A or B," "at least one of A and B," "at least one of A or B," "A, B, or C," "at least one of A, B, and C," and "at least one of A, B, or C," may include any one of items listed together with a corresponding phase, or all possible combinations of the phrases.

The term "and/or" includes a combination of a plurality of related elements or any of a plurality of related elements.

Terms such as "1st", "2nd", or "first" or "second" may be used to simply distinguish a corresponding element from other corresponding elements, and do not limit the corresponding elements in other aspects (e.g., in terms of importance or order).

In addition, terms such as "front surface", "rear surface", "upper surface", "lower surface", "side surface", "left side", "right side", "upper portion", and "lower portion" used in the present disclosure are defined based on the drawings, and the shape and position of each element are not limited by these terms.

Terms such as "include" or "have" are intended to designate the presence of features, numbers, steps, operations, components, parts, or combinations thereof described in the present disclosure, and do not preclude the possibility of the presence or addition of one or more other features, numbers, steps, operations, components, parts, or combinations thereof.

When an element is referred to as being "connected", "coupled", "supported", or "contacted" with another element, this includes not only the case where the elements are directly connected, coupled, supported, or contacted, but also the case where the elements are indirectly connected, coupled, supported, or contacted through a third element.

When an element is referred to be positioned "on" another element, this includes not only the case where the element is in contact with the other element, but also the case where another element exists between the two elements.

In the present disclosure, the "size" of particles refers to the "particle diameter" of particles unless otherwise defined.

In the present disclosure, the "particle diameter" of particles refers to an average diameter when the particles are spherical, and refers to an average major axis length when the particles are non-spherical. The particle diameter of particles may be measured by using a particle size analyzer (PSA). The "particle diameter" of particles is, for example, an "average particle diameter". The "average particle diameter" is the median particle diameter (D50) unless explicitly stated otherwise. The median particle diameter D50 refers to the size of particles corresponding to a cumulative value of 50 %, calculated from a particle side having the smallest particle size in a cumulative distribution curve of particle sizes in which particles are accumulated in the order of particle sizes from the smallest particle to the largest particle. The cumulative value may be, for example, a cumulative volume. The median particle diameter D50 may be measured by, for example, a laser diffraction method. Alternatively, the "average particle diameter" may be measured by a software or manual using a scanning electron microscope (SEM) image or a transmission electron microscope (TEM) image.

In the present disclosure, a "refrigerator" is a home appliance in which cooling air generated by a compressor of a cooling supplying device is supplied to a storage chamber, enabling the long-term fresh preservation of various foods.

The refrigerator may include, for example, a main body, and the main body may include an inner case and an outer case disposed outside the inner case. The refrigerator may include, for example, an insulator provided between the inner case and the outer case.

In the present disclosure, the "inner case" is a member forming a storage compartment. The inner case may include, for example, a case, a panel, a panel, or a liner. The inner case may be formed, for example, as one body or may be formed by assembling a plurality of plates.

In the present disclosure, the "outer case" is a member that forms an appearance of the main body. The outer case may be coupled to the outer side of the inner case such that an insulation material is disposed between the inner case and the outer case.

In the present disclosure, the "storage chamber" is a space for storing articles in the refrigerator. The storage chamber may include, for example, a space defined by the inner case. The storage chamber may further include, for example, an inner case defining a space corresponding to the storage chamber. The storage chamber may store, for example, various articles such as food, drugs, cosmetics, and the like, and the storage chamber may be formed to have at least one side open in order to take in and take out the articles.

The refrigerator may include, for example, one or more storage chambers. When two or more storage chambers are formed in the refrigerator, each storage chamber may have different uses and may be maintained at different temperatures. To achieve this, the storage chambers may be partitioned from each other by a partition wall including an insulating material.

The storage chamber may be, for example, provided to be maintained in an appropriate temperature range depending on the purpose, and may include "a fridge compartment," "a freezing compartment," and/or "a convertible compartment" divided according to the purpose and/or the temperature range. The fridge compartment may be, for example, maintained at a temperature appropriate for refrigerating and storing an item. The freezing compartment may be, for example, maintained at a temperature suitable for freezing and storing an item.

In the present disclosure, the "refrigeration" refers to cooling an item to the extent that it does not freeze. For example, the fridge compartment may be maintained at a temperature in a range of 0 °C to 7 °C.

In the present disclosure, "freezing" refers to cooling an item to freeze it or maintain it in a frozen state. For example, the freezing compartment may be maintained at a temperature in a range of -20 °C to -1 °C.

The convertible compartment may be used as any one of the fridge compartment and the freezing compartment, according to choice of a user or regardless of a user.

The storage chamber may be referred to as various names such as "vegetable compartment," "fresh compartment," "cooling compartment," and "ice-making compartment" in addition to the names of "fridge compartment," "freezing compartment," and "convertible compartment." The terms "fridge compartment," "freezing compartment," and "convertible compartment" used below should be each understood as encompassing a storage chamber having corresponding use and temperature ranges.

The refrigerator may include at least one door configured to open and close the open side of the storage chamber.

In the present disclosure, a "door" is a member configured to open and close one storage chamber or each of one or more storage chambers, or to open and close a single door for multiple storage chambers. The door may be installed on the front of the main body to rotate or slide.

The door may be, for example, configured to seal the storage chamber when the door is closed. The door may include, for example, an insulation material, similar to the main body, to insulate the storage chamber when the door is closed.

The door may include, for example, a door outer plate forming a front surface of the door, a door inner plate forming a rear surface of the door and facing the storage chamber, an upper cap, a lower cap, and a door insulator provided inside the foregoing.

For example, around the edge of the door inner plate, a gasket may be provided to seal the storage chamber by tightly fitting against the front surface of the main body when the door is closed. The door inner plate may include, for example, a dyke that protrudes rearward to accommodate a door basket for storing an item.

The door may include, for example, a door body and a front panel that is detachably coupled to the front side of the door body and forms the front surface of the door. The door body may include, for example, a door outer plate forming a front surface of the door body, a door inner plate forming a rear surface of the door body and facing the storage chamber, an upper cap, a lower cap, and a door insulation material provided inside the foregoing.

The refrigerator may be, for example, classified into a French door type, a side-by-side type, a bottom mounted freezer (BMF), a top mounted freezer (TMF), or a one-door refrigerator, depending on the arrangement of the door and the storage chamber.

The refrigerator may include a cooling air supplying device provided to supply cooling air to the storage chamber.

In the present disclosure, the term "cooling air supplying device" refers to a machine, a tool, an electronic device, and/or a system configured to generate cooling air and guide the cooling air to cool the storage chamber.

The cooling air supplying device may generate cooling air through a refrigeration cycle including compression, condensation, expansion, and evaporation processes of a refrigerant. To this end, the cooling air supplying device may include a compressor, a condenser, an expansion device, and an evaporator capable of driving the refrigeration cycle.

The refrigerator may include a machine room provided to arrange at least some components belonging to the cooling air supplying device.

In the present disclosure, a "machine room" refers to a space where at least some components belonging to the cooling air supplying device or the like are arranged. In order to prevent heat generated from the components arranged in the machine room from being transferred to the storage chamber, the machine room may be partitioned and insulated from the storage chamber. To dissipate heat from the components arranged in the machine room, the interior of the machine room may be configured to be connected with the outside of the main body.

The refrigerator may further include a dispenser provided on a door to provide water and/or ice. The dispenser may be provided on a door so that a user may access the refrigerator without opening the door.

The refrigerator may include an ice-making device provided to generate ice. The ice-making device may include an ice-making tray for storing water, an ice-discharging device for separating ice from the ice-making tray, and an ice bucket for storing ice generated by the ice-making tray.

In the present disclosure, an "air purifier" is a home appliance that sucks contaminated air from an indoor space, removes dust, odors, and the like contained in the air, and then discharges purified air thus obtained to the outside.

The air purifier includes, for example, a main body, and the main body includes an inlet and an outlet.

In the present disclosure, the "inlet" is an opening that introduces contaminated air from an indoor space into a main body. The inlet is an opening, a structure, or a system, through which fluid is introduced into the main body due to a pressure difference between the outside and the inside. The flow rate passing through the inlet depends on the pressure difference, fluid properties, inlet shape, and the like.

In the present disclosure, the "outlet" is an opening through which purified air is discharged into the indoor space outside the main body. The outlet is an opening, a structure, or a system, through which fluid is discharged to the outside of the main body due to a pressure difference between the outside and the inside. The flow rate passing through the outlet depends on the pressure difference, fluid properties, outlet shape, and the like.

The air purifier includes a fan and a fan motor for driving the fan, within the main body.

In the present disclosure, the "fan" is a member that moves air introduced into the main body through the inlet so that the air is discharged to the outside of the main body through the outlet. The fan is a member that rotates a fan blade connected to a rotation axis to cause the movement of the air.

In the present disclosure, the "fan motor" is a member that is connected to the rotation axis of the fan to provide a rotational force for rotating the fan blade.

The air purifier may include a sensor for detecting a state of the air purifier.

In the present disclosure, the "sensor" may detect one or more of a current and a voltage input to the fan motor, wind velocity at the outlet side, wind velocity at the inlet. The sensor may include, for example, a fan motor sensor, a wind speed sensor, and the like. The fan motor sensor may be arranged at a position for measuring current and voltage that are input and output to the fan motor. The wind velocity sensor is arranged around the outlet to detect wind velocity on the outlet side.

The air purifier may include an input unit, a display unit, and an output unit for operating the air purifier.

In the present disclosure, the "input unit" may be provided outside the main body to receive various control commands from the user. Each input button of the input unit may be a push switch and a membrane switch that generate an input signal through pressure applied by the user, or may be a touch switch that generates an input signal through touch by a body part of the user.

In the present disclosure, the "display unit" is a configuration for displaying an operation state of the air purifier. The display unit may display, for example, the quality of indoor air quantitatively or qualitatively.

In the present disclosure, the "output unit" is a configuration for displaying an operation state of the air purifier in the form of a sound. The output unit may output, for example, air quality in the form of a guide voice, a melody, or the like.

In the present disclosure, the "air conditioner" is a home appliance that sucks air from an indoor space, cools the sucked air, and then discharges the cooled air thus obtained to the outside.

The air conditioner includes, for example, a main body, and the main body includes an inlet and an outlet.

In the present disclosure, the "inlet" is an opening that introduces indoor air into the inside of the main body.

In the present disclosure, the "outlet" is an opening through which cooled air is discharged into the indoor space outside the main body.

The air conditioner includes a fan and a fan motor arranged within the main body.

In the present disclosure, the "fan" is a member that moves air introduced into the main body through the inlet so that the air is discharged to the outside of the main body through the outlet. The fan is a member that rotates a fan blade connected to a rotation axis to cause the movement of the air.

In the present disclosure, the "fan motor" is a member that is connected to the rotation axis of the fan to provide a rotational force for rotating the fan blade.

The air conditioner includes a heat exchanger configured to exchange heat between air introduced through the inlet and a refrigerant.

In the present disclosure, the "heat exchanger" is a system used to transfer heat between a heat source and working fluid. The heat exchanger is used during a cooling process. The working fluid, that is, the refrigerant, is, for example, separated into rigid walls to prevent mixing with a heat source, that is, the introduced air.

The refrigerator, the air purifier, or the air conditioner may each include a processor for controlling the same.

In the present disclosure, the "processor" controls overall operation of the refrigerator, the air purifier, or the air conditioner. The processor refers to a hardware chip including an integrated circuit in which an electrical circuit is integrated. The processor may control the components of the refrigerator, the air purifier, or the air conditioner by executing a program stored in the memory. The processor may include a separate NPU that performs the operations of an artificial intelligence model. In addition, the processor may include a central processing unit, a graphics processing unit (GPU), and the like. The processor may generate, for example, a control signal for controlling the operations of the cooling air supplying device. For example, the processor may receive temperature information of the storage chamber from a temperature sensor, and may generate a cooling air control signal for controlling the operation of the cooling air supplying device based on the temperature information of the storage chamber.

The processor may process user input of a user interface according to a program and/or data memorized/stored in the memory, and may control an operation of the user interface. The user interface may be provided by using an input interface and an output interface. The processor may receive user input from the user interface. The processor may transmit a display control signal for displaying an image on the user interface and image data to the user interface, in response to the user input.

In the present disclosure, the "memory" may be configured to store or record various information, data, commands, programs, and the like necessary for the refrigerator, the air purifier, or the air conditioner to operate. The memory refers to a hardware chip including an integrated circuit in which an electrical circuit is integrated. The memory may be configured to store temporary data generated while generating a control signal for controlling components included in the refrigerator, the air purifier, or the air conditioner. The memory may include at least one of a volatile memory or a nonvolatile memory, or a combination thereof. The processor and the memory may be integrally provided or separately provided. The processor may include one or more processors. For example, the processor may include a main processor and at least one sub-processor. The memory may include one or more memories.

The refrigerator, the air purifier, or the air conditioner may include, for example, a processor and a memory for controlling all of the components included in each of the foregoing, and may include a plurality of processors and a plurality of memories for individually controlling each of the components. For example, the refrigerator may include a processor and a memory that control the operation of the cooling air supplying device according to output of the temperature sensor. The refrigerator may separately include a processor for controlling the operation of the user interface according to user input and a memory.

In the present disclosure, a "controller" refers to a part including the memory for storing or memorizing a program and/or data for controlling the refrigerator, the air purifier, or the air conditioner, and a processor for outputting a control signal for controlling the cooling air supplying device, the fan motor, the heat exchanger, and the like according to the program and/or data memorized in the memory.

A communication module may communicate with external devices such as a server, a mobile device, or other home appliances through a nearby access point (AP). The AP may connect a local area network (LAN) to which the refrigerator, the air purifier, the air conditioner, or a user device is connected to a wide area network (WAN) to which a server is connected. The refrigerator, the air purifier, the air conditioner, or the user device may be connected to a server through the WAN.

The input interface may include a key, a touch screen, a microphone, and the like. The input interface may receive the user input and transmit it to the processor.

The output interface may include a display, a speaker, and the like. The output interface may output various notifications, messages, information, and the like generated by the processor.

Hereinafter, a home appliance according to an embodiment of the present disclosure, such as a refrigerator, an air purifier, and an air conditioner including a deodorizing filter will be described with reference to the drawings.

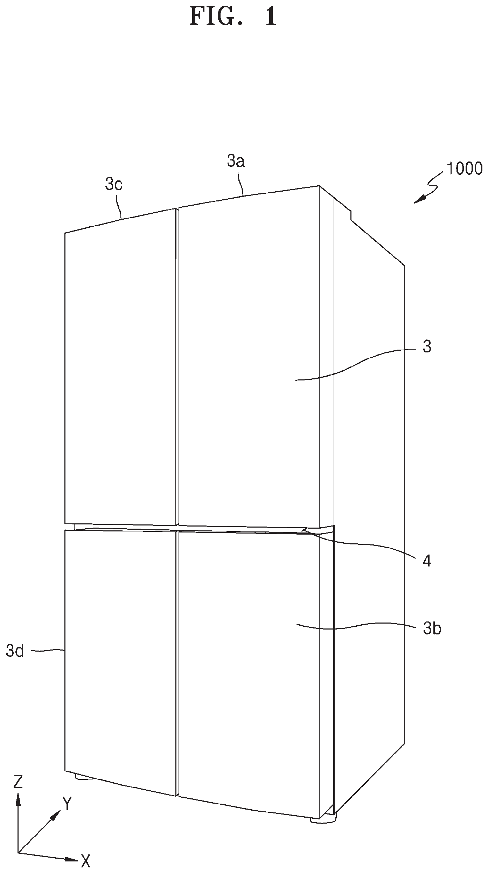

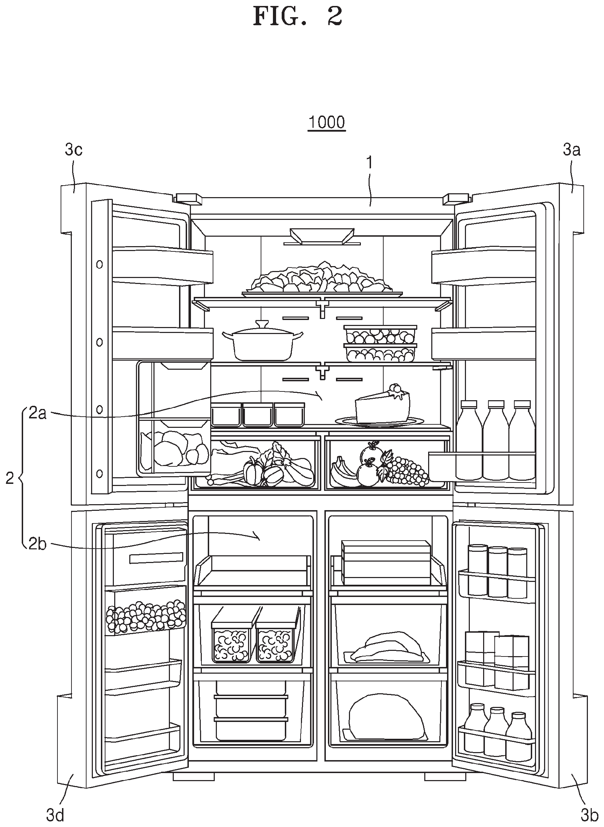

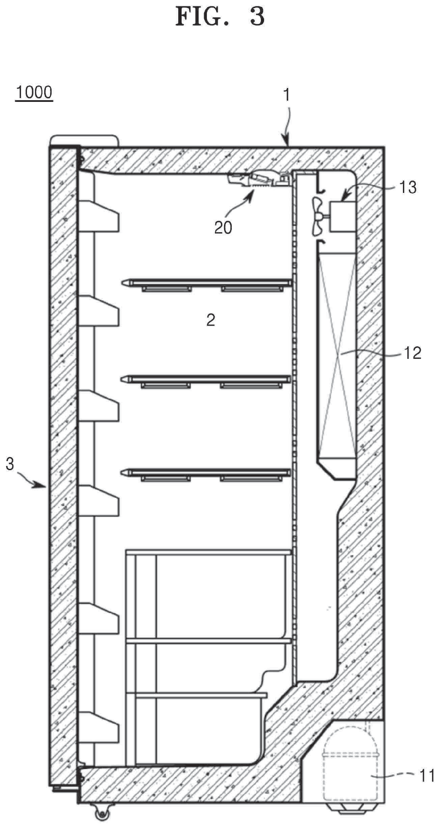

FIG. 1 is a perspective view of a refrigerator according to an embodiment of the present disclosure. FIG. 2 is a front view illustrating the inside of a refrigerator according to an embodiment of the disclosure. FIG. 3 is a cross-sectional view of a refrigerator according to an embodiment of the disclosure.

Referring to FIGS. 1 and 2, a refrigerator 1000 according to an embodiment of the present disclosure includes a main body 1 having storage chambers 2, 2a, and 2b, and doors 3, 3a, 3b, 3c, and 3d coupled to the main body 1 to open and close the storage chambers 2, 2a, and 2b. The main body 1 of the refrigerator 1000 includes: an inner case, and an outer case arranged outside the inner case. The refrigerator 1000 includes doors 3, 3a, 3b, 3c, and 3d configured to open and close one open side of the storage chamber 2. In the refrigerator 1000, the door 3 is illustrated to have four doors, but the number of the door 3 is not limited thereto. An upper door 3a and a lower door 3b on the right side of the refrigerator 1000 may be configured as one door, and an upper door 3c and an lower door 3d on the left side of the refrigerator 1000 may be configured as one door. In addition, the refrigerator 1000 may have more or fewer than four doors. In addition, the position of the door 3 may also be varied. A handle zone 4, which is a separation space where a user may put his/her hand to open and close the door 3, may exist between a plurality of doors 3a, 3b, 3c, and 3d. The door 3 may be configured to seal the storage chamber 2 when the door 3 is closed.

Referring to FIG. 3, the refrigerator 1000 according to an embodiment of the present disclosure includes the main body 1 having the storage chamber 2 and the door 3 coupled to the main body 1 to open and close the storage chamber 2. The refrigerator 1000 includes components for the freezing cycle, such as a compressor 11, a **condenser** (not shown), an evaporator 12, and an expansion valve (not shown). Cooling air generated by the evaporator 12 is supplied to the storage chamber 2 through an air blower 13 to maintain the storage chamber 2 at a low temperature. To remove odors generated in food and the like stored in the storage chamber 2, the refrigerator 1000 includes a deodorizing device 20. The deodorizing device 20 is arranged on the inner surface of the storage chamber 2. The deodorizing device 20 may be, for example, installed on the rear side of the upper surface of the storage chamber 2. The deodorizing device 20 includes a deodorizing filter (not shown). The deodorizing device 20 includes: for example, a housing (not shown) forming a flow path through which air passes; a deodorizing filter (not shown) arranged within the flow path inside the housing (not shown) to remove odors; and a fan (not shown) allowing air in the storage chamber 2 to pass through the flow path inside the housing (not shown) and the deodorizing filter (not shown) arranged within the flow path. The deodorizing device 20 may further include a filter light source (not shown) that irradiates light to the deodorizing filter to regenerate the deodorizing filter. The filter light source may, for example, irradiate ultraviolet rays to regenerate the deodorizing filter.

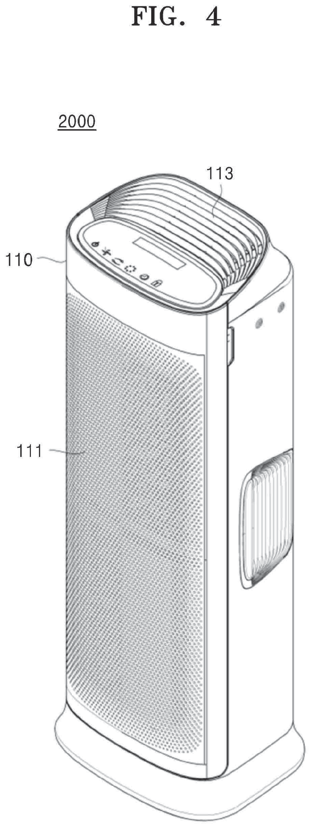

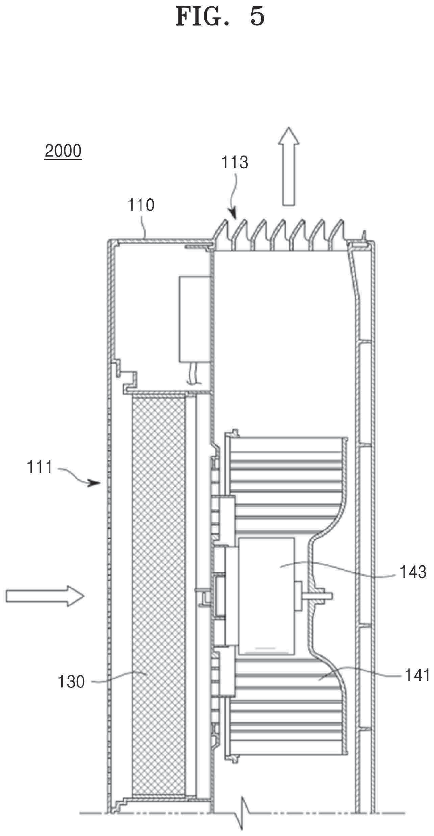

FIG. 4 is a perspective view illustrating the exterior of an air purifier according to an embodiment of the present disclosure. FIG. 5 is a cross-sectional view showing the inside of an air purifier according to an embodiment of the present disclosure.

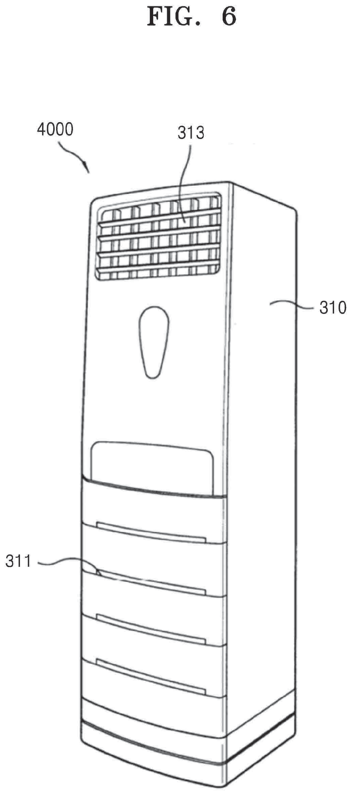

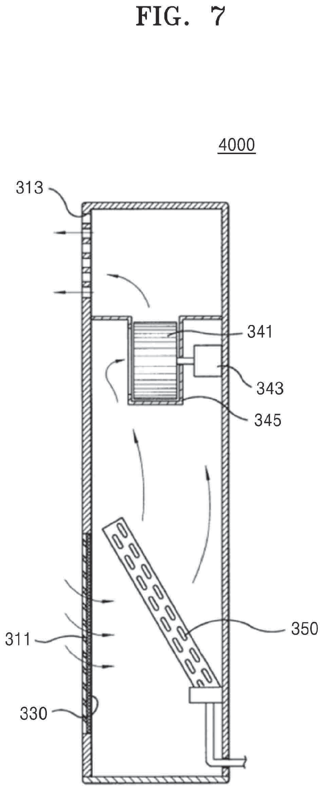

Referring to FIGS. 4 and 5, an air purifier 2000 includes a main body 110 forming the exterior, an inlet 111 for suctioning air from an indoor space, an outlet 113 through which the air introduced and purified is discharged, a fan 141 for moving air so that the air the air that has been introduced through the inlet 111 is discharged through the outlet 113, and a deodorizing device 130 arranged between the inlet 111 and the outlet 113. The deodorizing device 130 may be, for example, arranged adjacent to the inlet 111. The deodorizing device 130 may be, for example, arranged upstream of a fan 141. The inside of the main body 110 of the air purifier 2000 includes the deodorizing device 130, the fan 141, and a fan motor 143. The fan 141 is included to draw indoor air into the main body 110 through the inlet 111. The fan motor 143 is included to drive the fan 141. The fan 141 is driven by the fan motor 143, drawing indoor air into the main body 110 through the inlet 111. The air introduced into the inlet 111 passes through the deodorizing device 130, and odors in the air are then removed through the deodorizing device 130. The deodorizing device 130 includes a deodorizing filter (not shown). FIG. 6 is a perspective view of the exterior an air conditioner according to an embodiment of the present disclosure. FIG. 7 is a cross-sectional view of the interior of an air conditioner according to an embodiment of the present disclosure.

Referring to FIGS. 6 and 7, an air conditioner 4000 includes a main body 310 forming the exterior, an inlet 311 for drawing air from indoor space, an outlet 313 through which the air that has been introduced and then cooled is discharged, a fan 341 for moving air so that the air that has been introduced through the inlet 311 is discharged through the outlet 313, a heat exchanger 350 for exchanging heat between the air that has been introduced through the inlet 311 and a refrigerant, and a deodorizing device 330 arranged between the inlet 311 and the outlet 313. The deodorizing device 330 may be, for example, arranged adjacent to the inlet 311. The deodorizing device 330 may be, for example, arranged upstream of the heat exchanger 350. The deodorizing device 330 may be, for example, arranged upstream of the fan 341. The air that has been introduced through the inlet 311 at the lower end of the main body 310 is cooled by exchanging heat with the refrigerant of the heat exchanger 350, and then discharged through the outlet 313 at the upper end of the main body 310. The fan 341 is arranged at the upper end, that is, downstream of the heat exchanger 350. The fan 341 is surrounded by a duct 345. The fan 341 is driven by a driving motor 343. The fan 341 is connected to a shaft of the driving motor 343. The duct 345 surrounds side surfaces of the fan 341, and a front surface of the fan 341 is opened. The cooled air that has been introduced into the front surface of the fan 341 is moved to the opened upper portion of the duct 345 while rotating along the side surfaces of the fan 341. The cooled air moved to the opened upper portion of the duct 345 is discharged through the outlet 313 at the upper end of the main body 310. The air that has been introduced through inlet 311 passes through the deodorizing device 330, and odors in the air are then removed through the deodorizing device 330. The deodorizing device 330 includes a deodorizing filter (not shown).

The deodorizing device 20 of the refrigerator 1000 of FIGS. 1 to 3, the deodorizing device 130 of the air purifier 2000 of FIGS. 4 and 5, and the deodorizing device 330 of the air conditioner 4000 of FIGS. 6 and 7 each include a deodorizing filter (not shown).

The deodorizing filter includes: a substrate; and a deodorizing film on a surface of the substrate. The deodorizing film includes a deodorizing catalyst. The deodorizing filter contains δ-type MnO₂ and CeO₂, and has a structure in which the CeO₂ is coated with the δ₋type MnO₂. Due to the structure in which the CeO₂ is coated with the δ₋type MnO₂, the deodorizing catalyst may have an increased specific surface area. Consequently, the deodorizing catalyst may have improved deodorization performance. Meanwhile, a simple blend of the CeO₂ and the δ-type MnO₂ may reduce the specific surface area of the deodorizing catalyst as the CeO₂ is arranged on a surface of the δ-type MnO₂. Consequently, the deodorizing catalyst may have degraded deodorization performance. In the structure in which the CeO₂ is coated with the δ-type MnO₂, the δ-type MnO₂ is strongly anchored to the CeO₂, thereby facilitating oxygen transfer between the δ-type MnO₂ and the CeO₂ more easily. Consequently, the regeneration effect of the δ-type MnO₂ is promoted, thereby improving the lifespan of the deodorizing catalyst.

### <Configuration of deodorizing filter according to embodiment of present disclosure>

### (1) Deodorizing filter

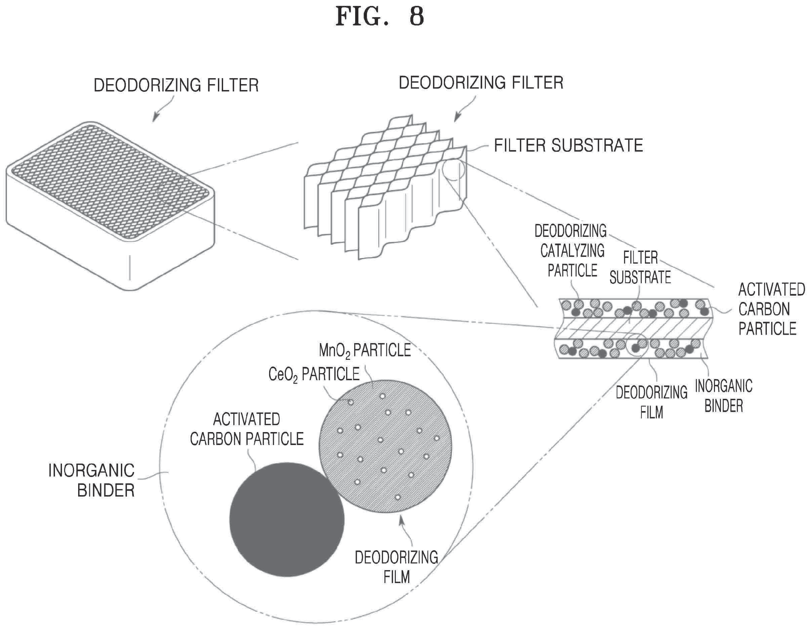

A deodorizing filter of the present embodiment is installed in an air purifier, a refrigerator, or an air conditioner to adsorb and decompose substances that cause odors, for example, formaldehyde, ammonia, toluene, trimethylamine, methylmercaptan, and the like. Specifically, such a deodorizing filter may be, as shown in FIG. 8, a honeycomb filter including a filter substrate constituting a honeycomb structure and a deodorizing film formed on a surface of the filter substrate and exhibiting a deodorizing function. In addition, the deodorizing filter is not limited to the honeycomb filter, and may be of any shape as long as the deodorizing film is formed on the surface of the substrate.

### (2) Deodorizing film

The deodorizing film may be a thin film containing a particulate deodorizing catalyst and an inorganic binder for supporting the deodorizing film onto the filter substrate. The deodorizing catalyst of the present embodiment is a polar material including a metal oxide as a main substance, and exhibits adsorption properties against formaldehyde, ammonia, trimethylamine, methylmercaptan, and the like, which are polar materials among odor molecules. In addition, the deodorizing catalyst of the present embodiment has a decomposition function without heating the aforementioned polar material.

When the content of the deodorizing catalyst in the deodorizing film is too small, the deodorizing catalyst may become buried within the inorganic binder, potentially degrading adsorption and decomposition abilities. For example, based on 100 wt% of the total weight of the deodorizing film, the content of the deodorizing catalyst may be 50 wt% or more or 80 wt% or more.

On the other hand, when the content of the deodorizing catalyst in the deodorizing film is too large, the deodorizing catalyst may not be able to be dispersed in the inorganic binder. For example, based on 100 wt% of the total weight of the deodorizing film, the content of the deodorizing catalyst may be 98 wt% or less or 97 wt% or less.

The inorganic binder may consist of, for example, colloidal silica, water glass, calcium silicate, alumina sol, titania sol, silicon oil, metal alkoxide, and the like, but is not limited thereto.

The deodorizing film may further contain activated carbon. The activated carbon may adsorb and retain a non-polar material which is difficult for a polar substance such as the deodorizing catalyst to adsorb. The non-polar material may include, for example, toluene or the like. In the deodorizing film, particles of the activated carbon may be present so as to be adjacent to particles of the deodorizing catalyst. To this end, instead of physically mixing activated carbon with δ-type MnO₂, as in the case of CeO₂ particles, activated carbon coated with δ-type MnO₂ by a crystallization method may be used.

When the content of the activated carbon is too small, the non-polar material may not be adsorbed and retained, and thus may be re-released before decomposition. For example, based on 100 wt% of the total weight of the deodorizing film, the content of the activated carbon may be 5 wt% or more or 10 wt% or more.

On the other hand, when the content of the activated carbon is too large, the amount of odor molecules that the deodorizing catalyst may adsorb decreases. This results in insufficient ability of decomposing the odor molecules, thereby causing breakthrough over time and potentially releasing odors. For example, based on 100 wt% of the total weight of the deodorizing film, the content of the activated carbon may be 20 wt% or less or 15 wt% or less.

The deodorizing film may contain, for example, at least one metal oxide selected from MgO, FeO, Fe₂O₃, ZrO₂, CuO, NiO, Mn₃O₄, Co₃O₄, Al₂O₃, Y₂O₃, ZnO, MoO₃, IrO₂, WO₃, W₂O₃, and TiO₂. As the deodorizing film contains such a metal oxide, the type of odor molecules that may be decomposed may be broaden and the decomposition performance of odor molecules may be further improved.

### (3) Deodorizing catalyst

The deodorizing catalyst is a particulate deodorizing catalyst containing δ-type manganese dioxide (MnO₂) and cerium oxide (CeO₂) constituting a layered structure.

In the deodorizing catalyst of the present embodiment, the δ-type MnO₂ is synthesized by growing crystals on the surface of CeO₂ particles. Accordingly, the CeO₂ particles form a structure coated with the δ-type MnO₂, and the CeO₂ and the δ-type MnO₂ are then strongly bonded to each other through an anchoring effect. Based on the structure in which the CeO₂ particles are embedded within the δ-type MnO₂, the CeO₂ and the δ-type MnO₂ are strongly adhered to each other through an anchoring effect.

The deodorizing catalyst of the present embodiment contains the δ-type MnO₂ having a layered structure with a large interlayer distance, making it easier to introduce odor molecules that cause odor, as compared with a deodorizing catalyst containing α-type MnO₂ with small pore diameters of a tunnel-like structure, thereby facilitating adsorption of such odor molecules. By containing the CeO₂, the deodorizing catalyst of the present embodiment may supply oxygen to the δ-type MnO₂ responsible for the catalytic action, and may accordingly regenerate the catalytic action of the δ-type MnO₂. The deodorizing catalyst of the present embodiment has a structure in which the CeO₂ is coated with the δ-type MnO₂ (CeO₂ embedded within δ-type MnO₂), and since the CeO₂ and the δ-type MnO₂ are in close contact through an anchoring effect, oxygen transfer from the CeO₂ to δ-type MnO₂ may occur easily. The deodorizing catalyst of the present embodiment has a structure in which the CeO₂ is coated with the δ-type MnO₂, and since the CeO₂ and the δ-type MnO₂ are in close contact through an anchoring effect, the CeO₂ is difficult to detach from the δ-type MnO₂, and thus the regeneration of the catalytic function of the δ-type MnO₂ is promoted, thereby maintaining the catalytic performance over a long period of time. The δ-type MnO₂ having high BET facilitates oxygen supply deep into the MnO₂. Thus, coating the CeO₂ with the δ-type MnO₂ may enable oxygen exchange not only at the surface of the _{δ-}type MnO₂, but also within the interior of the _{δ-}type MnO₂, thereby promoting the regeneration of the catalytic action.

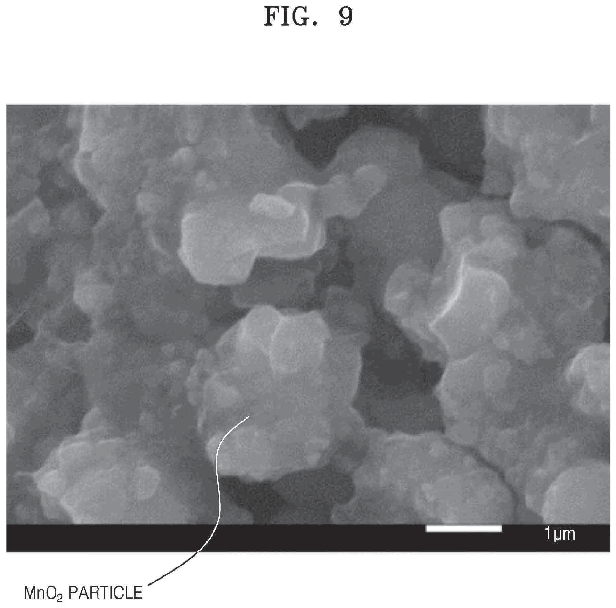

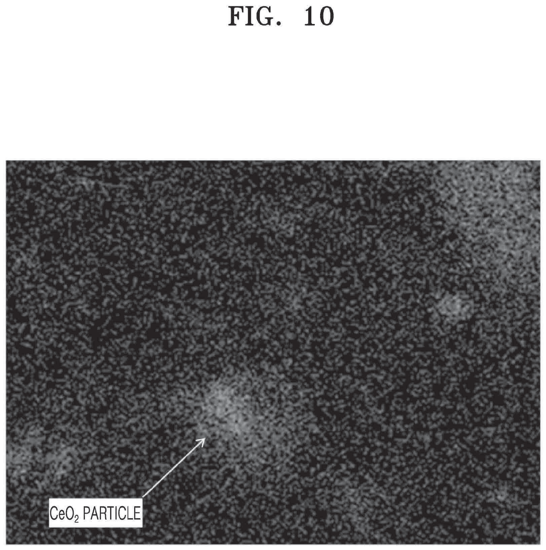

The structure in which the CeO₂ is coated with the δ-type MnO₂ may be confirmed by observation by SEM images and EDX mapping analysis performed on the deodorizing catalyst. An example thereof is shown in FIGS. 9 and 10. As shown in FIG. 9, the SEM image of the deodorizing catalyst of the present embodiment reveal only the δ-type MnO₂, with no detectable CeO₂. Meanwhile, EDX mapping analysis of the observation point in FIG. 9 confirms the presence of the CeO₂, as shown in FIG. 10. By comparing FIGS. 9 and 10, it may be confirmed that the CeO₂ is coated with the δ-type MnO₂.

The deodorizing catalyst of the present embodiment, which forms the structure in which the CeO₂ is coated with the δ-type MnO₂ (CeO₂ embedded within δ-type MnO2), may be obtained by pre-adding CeO₂ particles during a process of synthesizing the δ-type MnO₂ (a process of growing crystals of the δ-type MnO₂). Specifically, δ-type MnO₂ may be obtained by adding manganese sulfate (MnSO₄) dropwise into a potassium permanganate (KMnO₄) solution. By pre-adding CeO₂ particles to the KMnO₄ solution before adding the MnSO₄ dropwise, crystals of the δ-type MnO₂ may be grown on the surface of the CeO₂ particles. Accordingly, the deodorizing catalyst forming the structure in which the CeO₂ particles are coated with the δ-type MnO₂ (CeO₂ embedded within δ-type MnO₂) and containing the CeO₂ and the δ₋type MnO₂ that are strongly bonded to each other by an anchoring effect may be obtained.

By growing the δ₋type MnO₂ on the surface of the CeO₂ particle, rather than physically mixing δ-type MnO₂ particles with CeO₂ particles, a porous structure of the δ-type MnO₂ may be less susceptible to damage, thereby obtaining an excellent specific surface area as described below.

The total content of the δ-type MnO₂ and the CeO₂ in the catalyst is set at 100 wt%, and the preferable content of each substance is as follows.

When the content of the δ-type MnO₂ is too small, the surface area for adsorbing odor molecules is insufficient, resulting in inadequate deodorization and decomposition performance. For example, the content of the δ-type MnO₂ may be 90 wt% or more or 94 wt% or more.

When the content of the δ-type MnO₂ is too large, the supply of oxygen by the CeO₂ may be insufficient, preventing the catalyst function from being fully regenerated. For example, the content of the δ-type MnO₂ may be 99.5 wt% or less or 96 wt% or less.

When the content of the CeO₂ is too small, the supply of oxygen to the δ-type MnO₂ may be insufficient, preventing the catalyst function from being fully regenerated. For example, the content of the CeO₂ may be 0.5 wt% or more or 4 wt% or more.

When the content of the CeO₂ is too large, the surface area for adsorbing odor molecules may be insufficient, potentially leading to inadequate deodorization decomposition performance. For example, the content of the CeO₂ may be 10 wt% or less or 6 wt% or less.

A preferred average particle diameter of each particle in the deodorizing catalyst is as follows. The average particle diameter of each particle in the deodorizing catalyst may be measured from the particle size distribution by laser diffraction and scattering methods.

When the average particle diameter of the δ-type MnO₂ catalyst coating the CeO₂ is too large, it may be difficult to support catalyst particles by using an inorganic binder, which may cause powder shedding during use. For example, the average particle diameter of the δ-type MnO₂ catalyst coating the CeO₂ may be 20 µm or less or 10 µm or less. For example, the average particle diameter of the δ-type MnO₂ catalyst coating the CeO₂ may be 3 to 20 µm or 3 to 10 µm.

When the average particle diameter of the δ-type MnO₂ catalyst coating the CeO₂ is too small, the surface is covered with an inorganic binder as much as the increased catalyst surface area, preventing the catalyst from achieving sufficient deodorization performance. For example, the average particle diameter of the δ-type MnO₂ particles may be 3 µm or more.

When the average particle diameter of the CeO₂ is too large, the contact area with the δ-type MnO₂ decreases, preventing the catalyst from achieving sufficient oxygen supply performance. For example, the average particle diameter of the CeO₂ particles may be 0.5 µm or less. For example, the average particle diameter of the CeO₂ particles may be 0.01 to 0.5 µm.

The specific surface area of the deodorizing catalyst may be 250 m²/g or more, 280 m²/g or more, or 300 m²/g or more, as a BET value. The BET value of the specific surface area may be measured from gas adsorption amount by N₂. By setting the specific surface area of the deodorizing catalyst to 250 m²/g or more as the BET value, the deodorizing catalyst may have a sufficient surface area for adsorption of odor molecules, and may accordingly decompose odor molecules with high efficiency.

The δ₋type MnO₂ may have a layered structure. The distance between adjacent layers included in the layered structure may be, for example, 7 Å or more or 7.2 Å or more.

When the δ-type MnO₂ has an interlayer spacing of 7 Å or more, formaldehyde having a size of about 5.2 Å, ammonia having a size of 3.3 Å, toluene having a size of 5.9 Å, trimethylamine having a size of 7 Å, and methylmercaptan having a size of 4 Å may be easily adsorbed into the deodorizing catalyst and then decomposed.

The deodorizing catalyst may be regenerated by ultraviolet light. For example, an ultraviolet light source capable of regenerating the deodorizing catalyst may be additionally provided inside the refrigerator.

### Mode for the Invention

### Examples

Hereinafter, the present disclosure will be described in more detail with reference to Examples. The present disclosure is not limited to Examples below, and may be modified and implemented within a range that may be suitable for the purpose of the aforementioned and subsequent descriptions, and all such modifications are included within the technical scope of the present disclosure.

### (Experimental Example 1)

A deodorizing catalyst and a deodorizing film were prepared so as to be blended as shown in Table 1 (Examples 1 to 4 and Comparative Examples 1 to 5). In the column labeled blending in Table 1, "MnO₂:CeO₂" indicates a weight ratio of MnO₂ to CeO₂ in a deodorizing catalyst. "MnO₂," "CeO₂," and "activated carbon" each represent a weight ratio of the corresponding substance in a deodorizing film. Samples of Examples 1 to 4 and Comparative Examples 1 to 4 refer to a deodorizing film that includes a deodorizing catalyst containing δ-type MnO₂, and a sample of Comparative Example 5 refers to a deodorizing film that includes a deodorizing catalyst containing α-type MnO₂. The samples of Examples 1 to 4 and Comparative Examples 1 to 3 were obtained by growing δ-type MnO₂ crystals on the surface of CeO₂ particles according to the aforementioned method, and the sample of Comparative Example 4 was obtained by physically mixing previously CeO₂ particles and δ₋type MnO2 particles by using a mill or the like.

### (Analysis by SEM-EDX)

The surface of the deodorizing catalyst powder from each sample thus obtained was observed under a scanning electron microscope (SEM). In addition, by performing analysis via EDX mapping, the state of each particle was identified. In the deodorizing catalysts of Examples 1 to 4 and Comparative Examples 1 to 3 obtained by growing the δ-type MnO₂ crystals on the surface of the CeO₂ particles, it was confirmed by EDX mapping that, as shown in FIG. 10, the CeO₂ particles were embedded within the δ₋type MnO₂. FIG. 9 is an SEM image observing the same visual field as the EDX mapping shown in FIG. 10, showing the surface of the deodorizing catalyst of Example 4.

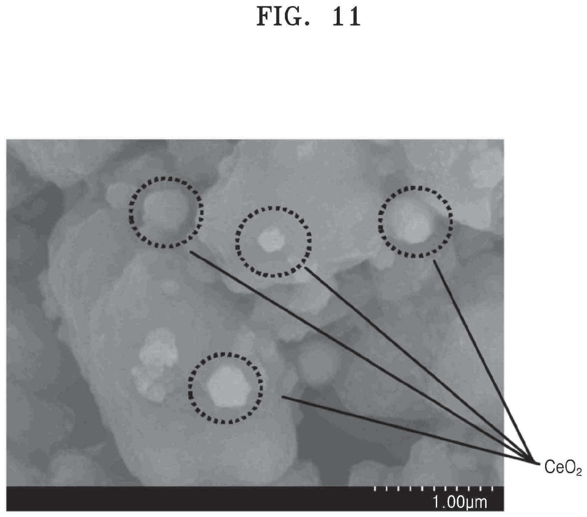

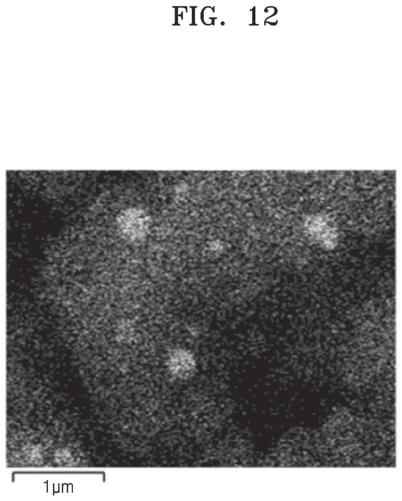

Meanwhile, in the deodorizing catalyst of Comparative Example 4 obtained by physically mixing the previously CeO₂ particles and δ-type MnO₂ particles by using a mill or the like, it was confirmed that the CeO₂ particles were not embedded within the δ-type MnO₂, but were rather attached to the surface of the δ-type MnO₂ particles as shown in the SEM image of FIG. 11. This appearance is also evident in FIG. 12 which shows the visual field of FIG. 11 observed via EDX mapping.

### (Measurement of blending)

The blending ratio of CeO₂ and MnO₂ in the deodorizing catalyst and the blending ratio of CeO₂, MnO₂, and activated carbon in the deodorizing film of each sample thus obtained were measured by performing quantitative analysis on Mn and Ce by using X-ray photoelectron spectroscopy (XPS) (KRATOS ULTRA2 manufactured by Shimadzu Corporation). The blending amount of the activated carbon was experimentally calculated based on the introduction amount.

### (Measurement of BET value)

The specific surface area (BET value) of each sample was measured by calculating from the adsorption curve within a range of the relative pressure (p/p0=0.0 to 0.20) of the N₂ adsorption isotherm measured at 298 K, by using a gas/vapor adsorption measuring device (MicrotacBEL Corp., BELSORP-max II). (Note: JIS Z 8830)

### (Measurement of particle diameter distribution)

The particle diameter distribution of the δ-type MnO₂ catalyst coating the CeO₂ of each sample was measured by image observation by using a laser diffraction type particle diameter distribution measuring device (manufactured by Shimadzu Corp., SALD-2300) and a scanning electron microscope (SEM) (manufactured by Nihon Denshi Co., Ltd., JSM-IT800).

For each sample thus obtained, evaluation of deodorization performance and lifespan was performed. The evaluation method is as follows.

### (Evaluation of deodorization performance)

The deodorization performance of a honeycomb filter including the deodorizing film of each sample formed on the surface was evaluated against odor molecules (formaldehyde, ammonia, and toluene) targeted by an air purifier filter and odor molecules (trimethylamine and methylmercaptan) targeted by a refrigerator filter.

Specifically, the filter was installed in a 500 L chamber, and after injecting a predetermined amount of measurement gas into the chamber, the deodorization performance was measured by using a detection tube for the measurement gas. For each odor molecule, the residual rate (%) was calculated as a ratio of the concentration after 60 minutes to the concentration after 0 minute (initial concentration), and the residual rate of 20 % or less was denoted as "○", the residual rate exceeding 20 % but less than 60 % was denoted as "△", and the residual rate of 60 % or more was denoted as "×". For the samples where both the odor molecules targeted by the air purifier and those targeted by the refrigerator were evaluated as "△" or "×", the comprehensive deodorization performance evaluation was rated "×", and for all other samples, the comprehensive deodorization performance evaluation was rated "○". The results are shown in Table 1.

### (Lifespan evaluation)

The lifespan of a honeycomb filter including the deodorizing film of each sample formed on the surface was evaluated. Specifically, the filter was installed in a 500 L chamber, and after injecting a predetermined amount of measurement gas into the chamber, a first deodorization rate was measured by using a detection tube for the measurement gas. After the test, as a regeneration operation, ventilation was performed on the filter for 1 hour by fan operation. This operation was set as one cycle, and was performed for 20 cycles. The lifetime was calculated as a ratio of the deodorizing rate (%) at the 20th cycle the deodorizing rate (%) at the 1st cycle. The value 60 % or more was denoted as "○", the value of exceeding 40 % but less than 60 % was denoted as "△", and the value of 40 % or less was denoted as "×". The results are shown in Table 1.

### (Overall judgment)

When both the deodorization performance evaluation and the lifespan evaluation were rated "○", the overall judgment was also rated "○ (good)". Meanwhile, when one of the deodorization performance evaluation and the lifespan evaluation was rated "△" or "×", the overall judgement was rated "×" (poor).

**[Table 1]**

| | Blending (wt%) | | | | BET (m²/g) | Particle diameter distribution (µm) | | Slurry dispersibility | Deodorization performance evaluation | | | | | | Lifespan evaluation | Overall judgement | Remark |
|---|---|---|---|---|---|---|---|---|---|---|---|---|---|---|---|---|---|
| | | | | | | | | | Air purifier | | | Refrigerator | | Deodorization performance evaluation | | | |
| | MnO₂ | CeO₂ | Activated carbon | MnO₂:CeO₂ | | MnO₂ | CeO₂ | | HCHO | NH₃ | Toluene | TMA | MM | | | | |
| Example1 | 84.6 | 5.4 | 10 | 94:6 | 258 | 4~5 | 0.2~0.4 | ○ | ○ | ○ | ○ | ○ | ○ | ○ | ○ | ○ | Standard blending |
| Example2 | 88.2 | 1.8 | 10 | 98:2 | 321 | 4~5 | 0.2~0.4 | ○ | ○ | ○ | ○ | ○ | ○ | ○ | ○ | ○ | High Mn content |
| Example3 | 82.8 | 7.2 | 10 | 92:8 | 292 | 4~5 | 0.2~0.4 | ○ | ○ | ○ | ○ | ○ | ○ | ○ | ○ | ○ | Low Mn content |
| Example4 | 94 | 6 | 0 | 94:6 | 258 | 4~5 | 0.2~0.4 | ○ | ○ | ○ | × | ○ | ○ | ○ | ○ | ○ | No activated carbon |
| Comparative Example1 | 90 | 0 | 10 | 100:0 | 260 | 4~5 | - | ○ | ○ | ○ | ○ | ○ | ○ | ○ | × | × | No CeO₂ |
| Comparative Example2 | 76.5 | 13.5 | 10 | 85:15 | 190 | 4~5 | 0.2-0.4 | ○ | Δ | ○ | ○ | Δ | Δ | × | × | × | BET<250(low MnO₂) |
| Comparative Example3 | 84.6 | 5.4 | 10 | 94:6 | 305 | 21 | 0.2-0.4 | ○ | N/A | | | | | | | × | Mn particle diameter exceeding upper limit |
| Comparative Example4 | 84.6 | 5.4 | 10 | 94:6 | 214 | 4~5 | 0.2~0.4 | ○ | Δ | Δ | ○ | Δ | Δ | × | × | × | BET<250 (Physical mixing) |
| Comparative Example5 | 84.6 (α-MnO₂) | 5.4 | 10 | 94:6 | 281 | 4~5 | 0.2~0.4 | ○ | × | ○ | ○ | × | ○ | × | × | × | Different types of MnO₂ |

As shown from the results of Table 1, it was confirmed that the deodorizing filters (Examples 1 to 4), which were prepared by using the deodorizing catalyst having a structure in which the CeO₂ was embedded within the δ-type MnO₂, having an average particle diameter 20 µm or less, and having a specific surface area of 250 m²/g or more as a BET value, obtained excellent results in both the deodorization performance evaluation and the lifespan evaluation. The deodorizing filter of Example 4 was rated "×" for its deodorization performance against toluene. This is due to the deodorizing film not containing activated carbon which is a non-polar substance.

Meanwhile, in Comparative Examples 1 to 5, either or both of the deodorization performance evaluation and the lifespan evaluation failed to obtain excellent results.

In Comparative Example 1, the lifespan evaluation was rated "×", which was determined to be due to the lack of oxygen supply to the δ-type MnO₂, which adsorbed and decomposed odor molecules, because the catalyst did not contain CeO₂.

In Comparative Example 2, the deodorization performance evaluation was rated "×", which was determined to be due to the low δ-type MnO₂ content in the deodorizing catalyst, resulting in the overall specific surface area (BET value) of less than 250 m²/g.

In Comparative Example 3, the particle diameter of the δ-type MnO₂ in the prepared catalyst was too large, leading to poor slurry dispersibility and preventing the formation of a deodorizing film on the surface of the filter.

In Comparative Example 4, the deodorization performance evaluation was rated "×", which was considered to be due to the destruction of the porous structure of the δ-type MnO₂ by physically mixing the δ-type MnO₂ particles and the CeO₂ particles, causing the specific surface area (BET value) to decrease to 250 m²/g or less. In Comparative Example 4, the lifespan evaluation was rated "×", which was considered to be due to the failure to maintain the catalytic activity effect of the CeO₂. This is because, when physically mixing the δ-type MnO₂ particles and the CeO₂ particles, the δ-type MnO₂ particles and the CeO₂ particles did not firmly adhered to each other, and when slurried, the CeO2 particles were detached from the MnO₂ particles.

In Comparative Example 5, the deodorization performance evaluation was rated "×", which was considered to be due to the difficulty in capturing odor molecules when using α-type MnO₂ having a tunnel-type structure with a small pore diameter.

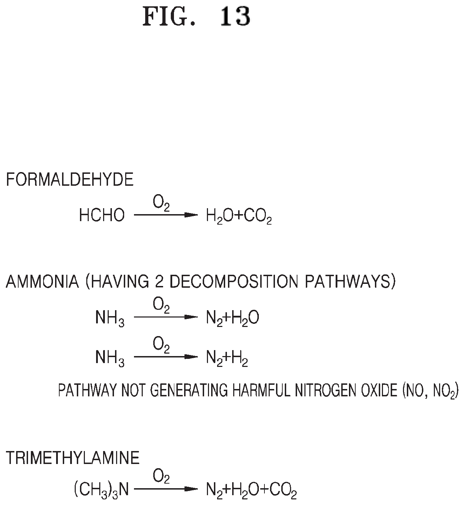

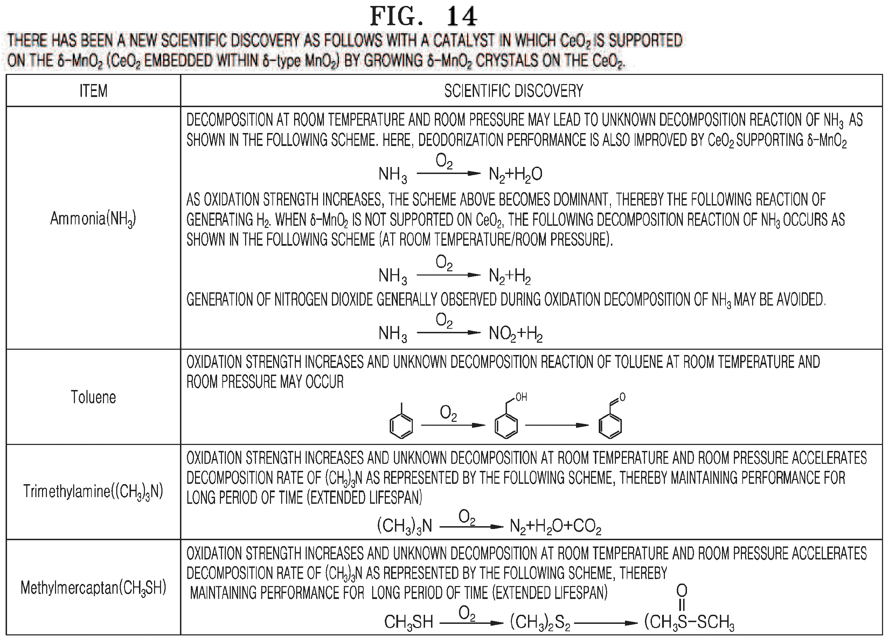

In the present experimental example, it became apparent that the decomposition of several odor molecules (specifically formaldehyde, ammonia, and trimethylamine) was carried out at room temperature without heating, via reaction schemes shown in FIG. 13 by using the δ-type MnO₂ coating the CeO₂. With the catalyst in which the CeO₂ was supported on the δ-type MnO₂ (the CeO₂ was embedded within the δ-type MnO₂) by growing the δ₋type MnO₂ crystals on the CeO₂, there has been a new finding on the principle of decomposition of odor molecules as shown in FIG. 14 (specifically ammonia, toluene, trimethylamine, and methylmercaptan). Details are shown in FIG. 14.

### (Experimental Example 2)

In Experimental Example 2, the effect of coating the CeO₂ with the δ-type MnO₂ on the deodorization performance against HCHO was confirmed.

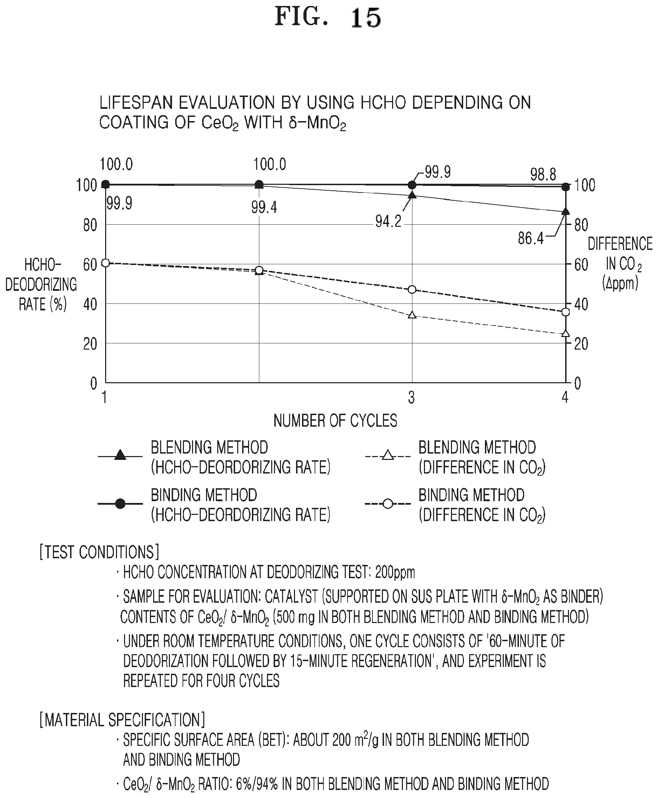

As shown in FIG. 15, two types of deodorizing catalyst samples having different preparation methods were prepared, and a deodorization test using HCHO was performed for each type. One type of the deodorizing catalyst sample was prepared by growing δ-type MnO₂ crystals on the surface of CeO₂ particles (described as "binding method"), and that is, CeO₂ was coated with δ₋type MnO₂. The other type of the deodorizing catalyst sample was obtained by physically mixing CeO₂ particles and δ-type MnO₂ particles by using a mill or the like (described as "blending method"), and that is, CeO₂ was not coated with δ₋type MnO₂. These two types of the catalyst particle samples have the same specific surface area, and also have the same weight ratio of CeO₂ to δ-type MnO₂. In addition, all deodorization tests were performed at the same temperature (room temperature). More specific test conditions and material specifications are as described in FIG. 15.

As shown in FIG. 15, it was confirmed that oxygen supply efficiency by the CeO₂ was improved by coating the CeO₂ with the δ-type MnO₂ under the conditions where the specific surface area is the same, the weight ratio of CeO₂ to δ-type MnO₂ is the same, and the test temperature is the same, and that the deodorizing rate and the decomposition action of HCHO exhibited high performance even after repeated tests.

### (Experimental Example 3)

In Experimental Example 3, the performance of regeneration promotion by irradiating UV-C onto the catalyst particles was confirmed.

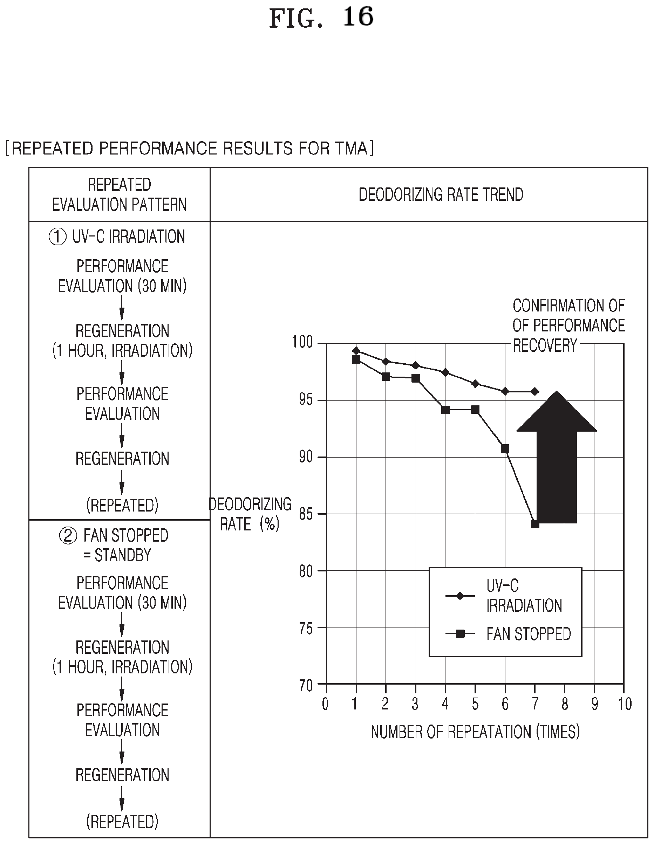

By using a honeycomb filter having the deodorizing film of the present disclosure formed on the surface, the effect of promoting decomposition by light irradiation was confirmed. For example, the deodorization performance against odor molecules (trimethylamine) targeted by the refrigerator filter was repeatedly evaluated by using a UV-C lamp (Sankyo Denki, 8 W). the filter was installed inside a 100 L chamber, and after injecting a predetermined amount of measurement gas into the chamber, the deodorization performance was measured by using a detection tube for the measurement gas. The deodorizing rate (%) was calculated as a ratio of the concentration after 30 minutes to the concentration after 0 minute (initial concentration) for trimethylamine, and the deodorizing rates obtained from repeated evaluations were plotted. Specific evaluation conditions and test results are shown in FIG. 16.

As shown in FIG. 16, after the performance evaluation, the deodorizing rate was compared with whether or not the UV-C irradiation was performed for 1 hour (with fan stopped as a common condition). In the evaluation carried out with 1 hour of UV-C irradiation, it was confirmed that deodorization performance tended to be maintained even when the deodorizing rate was repeated, and that performance recovery tended to occur as decomposition was promoted.

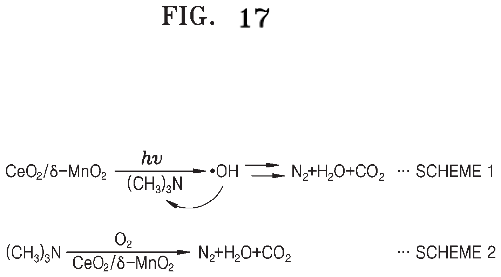

The current understanding of the principle by which irradiation with UV light promotes regeneration of the catalyst is shown in FIG. 17. The δ-type MnO₂ has a narrower band gap than other structures (α, β, and γ), and thus has high activity as a photocatalyst. That is, since the δ-type MnO₂ has high photocatalytic activity, ·OH may be generated by irradiating UV. The ·OH generated as shown in Scheme 1 of FIG. 17 reacts with trimethylamine (TMA) to proceed the decomposition. As shown in Scheme 2, the oxidation decomposition reaction at room temperature also proceeds via the same pathway, suggesting that the decomposition of TMA was promoted.

### Industrial Applicability

According to an embodiment, a refrigerator capable of exhibiting excellent deodorization performance over a long period of time with a deodorizing filter used in the refrigerator is provided.

According to an embodiment, an air purifier capable of exhibiting excellent deodorization performance over a long period of time with a deodorizing filter used in the air purifier is provided.

According to an embodiment, a home appliance capable of exhibiting excellent deodorization performance over a long period of time with a deodorizing filter used in the home appliance is provided.

## Claims

1. A refrigerator comprising:
an inner case forming a storage chamber;
a main body comprising an outer case on an outer side of the inner case;
a door configured to open and close the storage chamber; and
a deodorizing device arranged on an inner surface of the storage chamber,
wherein the deodorizing device comprises a deodorizing filter,
wherein the deodorizing filter comprises: a substrate; and a deodorizing film on a surface of the substrate,
wherein the deodorizing film comprises a deodorizing catalyst, and
wherein the deodorizing catalyst the deodorizing catalyst comprises δ-type MnO₂ and CeO₂,
wherein the deodorizing catalyst has a structure in which the CeO₂ is coated with the δ-type MnO₂,
wherein an average particle diameter of the δ-type MnO₂ is 20 µm or less, and
wherein the deodorizing catalyst has a specific surface area that is 250 m²/g or more as a BET value.

2. The refrigerator of claim 1, wherein
a content of the δ-type MnO₂ is 90 wt% to 99.5 wt%, and
a content of the CeO₂ is 0.5 wt% to 10 wt%.

3. The refrigerator of claim 1, wherein
the δ-type MnO₂ coating the CeO₂ has an average particle diameter of 20 µm or less.

4. The refrigerator of claim 1, wherein
the δ-type MnO₂ has a layered structure, and
a distance between adjacent layers included in the layered structure is 7Å or more.

5. The refrigerator of claim 1, wherein
the deodorizing film further comprises an inorganic binder.

6. The refrigerator of claim 1, wherein
the deodorizing film further comprises activated carbon.

7. The refrigerator of claim 1, wherein
the deodorizing film further comprises at least one selected from MgO, FeO, Fe₂O₃, ZrO₂, Cu-O, NiO, Mn₃O₄, Co₃O₄, Al₂O₃, Y₂O₃, ZnO, MoO₃, IrO₂, WO₃, W₂O₃, and TiO₂.

8. The refrigerator of claim 1, wherein
the substrate has a honeycomb structure.

9. The refrigerator of claim 1, further comprises
an ultraviolet light source for regenerating the deodorizing catalyst.

10. An air purifier comprising:
a main body comprising an inlet and an outlet;
a fan configured to transfer air introduced through the inlet, and configured to discharge the air through the outlet; and
a deodorizing device arranged between the inlet and the outlet,
wherein the deodorizing device comprises a deodorizing filter,
wherein the deodorizing filter comprises: a substrate; and a deodorizing film on a surface of the substrate,
wherein the deodorizing film comprises a deodorizing catalyst, and the deodorizing catalyst comprises δ-type MnO₂ and CeO₂,
wherein the deodorizing catalyst has a structure in which the CeO₂ is coated with the δ-type MnO₂,
wherein an average particle diameter of the δ-type MnO₂ is 20 µm or less, and
wherein the deodorizing catalyst has a specific surface area that is 250 m²/g or more as a BET value.

11. The air purifier of claim 10, wherein
a content of the δ-type MnO₂ is 90 wt% to 99.5 wt%, and
a content of the CeO₂ is 0.5 wt% to 10 wt%.

12. The air purifier of claim 10, wherein
an average particle diameter of the δ-type MnO₂ coating the CeO₂ is 20 µm or less.

13. The air purifier of claim 10, wherein
the deodorizing film further comprises
an inorganic binder or
activated carbon, or
further comprises at least one selected from MgO, FeO, Fe₂O₃, ZrO₂, CuO, NiO, Mn₃O₄, Co₃O₄, Al₂O₃, Y₂O₃, ZnO, MoO₃, IrO₂, WO₃, W₂O₃, and TiO₂.

14. The air purifier of claim 10, wherein
the substrate has a honeycomb structure.

15. A home appliance comprising:
a main body comprising an inlet and an outlet;
a fan configured to transfer air introduced through the inlet, and configured to discharge the air through the outlet;
a heat exchanger configured to exchange heat between the air introduced through the inlet and a refrigerant; and
a deodorizing device arranged between the inlet and the outlet,
wherein the deodorizing device comprises a deodorizing filter,
wherein the deodorizing filter comprises: a substrate; and a deodorizing film on a surface of the substrate,
wherein the deodorizing film comprises a deodorizing catalyst, the deodorizing catalyst comprises δ-type MnO₂ and CeO₂,
wherein the deodorizing catalyst has a structure in which the CeO₂ is coated with the δ-type MnO₂,
wherein an average particle diameter of the δ-type MnO₂ is 20 µm or less, and
wherein the deodorizing catalyst has a specific surface area that is 250 m²/g or more as a BET value.
